# EUROPEAN PATENT APPLICATION

(11) **EP 4 792 662 A1**
(43) Date of publication of application: **19.08.2026**
(21) Application number: 25764864.2
(22) Date of filing: 23.04.2025
(51) Int. Cl.: A23L 29/281, A23L 29/30, A23L 33/10, A23L 33/105, A23L 33/115, A61K 8/04, A61K 8/65

(54) **GEL COMPOSITION, PRODUCT CONTAINING SAME AND USE THEREOF**

(30) Priority: 17.12.2024 CN 202411855796
(71) Applicant: Sirio Pharma (Guangdong) Co., Ltd., Shantou Guangdong 515000 (CN)
(72) Inventor: ZHENG, Yirui, Shantou Guangdong 515000 (CN)
(74) Representative: Petraz, Gilberto Luigi
(86) International application number: PCT/CN2025/090577
(87) International publication number: WO 2026/129525

(57) **Abstract**

Provided in the present application are a gel composition, products containing the same, and use thereof. The gel composition includes gelatin, where, in the gel composition, a mass content of the gelatin is 4% to 13%, and a mass content of the lipid soluble component is not less than 10%; the weight ratio of gelatin to sugar alcohol is 0.12:1 to 0.4:1; an isoelectric point of the gelatin is I, and a pH of the gel composition after dilution to a mass concentration of 1% is H, and the following relationship within the gel composition is satisfied: 1 < I - H < 5.3.

## Description

### TECHNICAL FIELD

The present application relates to the field of gel compositions, and particularly relates to a gel composition, products containing the same, and use thereof.

### BACKGROUND

The stickiness on the surface of gel foods may cause problems of adhesion to packaging materials, thereby affecting the appearance integrity of the gel foods and further affecting the eating experience.

Specifically, in a first aspect, excessive surface stickiness of gel foods may cause them to stick together in the packaging materials, making it difficult to separate. In a second aspect, it may cause that a part of the food detaches and adheres to the packaging material, during the removal of the food from the inner packaging material, when the surface stickiness of the food is excessive high, meaning the product is unable to be completely separated from the packaging material. In a third aspect, certain components in the food may penetrate into the contact layer between the packaging material and the food due to affinity, which may cause the food to adhere to the packaging material.

In the prior art, it is possible to improve the surface tension of packaging materials by adjusting the type of packaging materials or adjusting the surface treatment methods of packaging materials, because the surface tension of packaging materials affects the wetting degree of the food. However, screening suitable packaging materials or synthesizing new packaging materials both require a long research and development time.

It is also possible to adjust the surface adhesiveness of gel foods to retain a complete appearance. In actual research processes, it is easy to confuse the two concepts of viscosity and surface stickiness of gel compositions. Viscosity refers to the physical quantity of the rheological properties exhibited by a gel composition in its fluid state that resists flow and quantifiably resists flow deformation; whereas the surface stickiness of a gel composition refers to its adhesive force toward other objects. The adhesion to the packaging material is greater when the surface stickiness of a gel food is high, meaning it is difficult to completely peel off the packaging material, thereby affecting the appearance integrity of the gel food.

In the prior art, adjusting the surface adhesiveness of gel foods is generally achieved by coating the surface of the gel food with sugar powder (i.e., "sugar sanding") to inhibit the surface adhesiveness of the gel food. However, during long-term storage or under high temperatures, the sugar powder may absorb moisture from the gel food, causing the sugar powder to become damp or melt, leading to issues such as changes in the texture of the gel food and adhesion of the sugar powder to the packaging material.

### SUMMARY

To improve the surface stickiness of gel foods so as to preserve the complete form of the gel food without affecting its texture and hardness, provided in the present application are a gel composition, products containing the same, and use thereof.

According to a first aspect of the present application, a gel composition is provided, comprising gelatin, a sugar alcohol, a lipid soluble component, and water. In the gel composition, a mass content of the gelatin is 4% to 13%, and a mass content of the lipid soluble component is not less than 10%. The weight ratio of gelatin to sugar alcohol is 0.12:1 to 0.4:1. An isoelectric point of the gelatin is I, and a pH of the gel composition after dilution to a mass concentration of 1% is H. Within the gel composition, the following relationship is satisfied: 1 < I - H < 5.3.

It was found by the inventor that the problem of the gel composition adhering to packaging materials may be improved either by reducing the surface stickiness of the gel composition or by increasing the cohesiveness of the gel composition. Cohesiveness refers to the tightness of the internal structure of the product. It indicates that the internal texture of the gel composition is denser when cohesiveness is higher, that is the gel composition has a better ability to maintain structural integrity; conversely, it indicates that the internal texture of the gel composition is looser. Specifically, in a case that the surface stickiness of the gel composition is relatively high, it may also reduce the risk of the gel composition adhering to the packaging material if the cohesiveness of the gel composition is sufficiently high. This is because the texture of the gel composition is dense; it may still completely separate from the packaging material even if the surface stickiness is relatively high. However, the risk of the gel composition adhering to the packaging material increases, and the possibility of preserving the complete form of the food decreases when the surface stickiness of the gel composition is relatively high but its cohesiveness is relatively low.

In the present application, the introduced gelatin is an amphoteric electrolyte extracted after hydrolysis of collagen, whose molecular chains contain both positively and negatively charged groups. As the content of gelatin in the gel composition increases, its cohesive force also increases; at the same time, the affinity between the protein in the gelatin and the contact layer of the packaging material also increases. Therefore, it may affect the adhesion between the gel food and the packaging material when the gelatin content is too high or too low, potentially damaging the complete form of the gel food. Furthermore, in addition to regulating the content of gelatin in the gel composition, the present application also introduces a sugar alcohol to counteract the affinity between gelatin protein and the packaging material. However, due to their polyhydroxy alcohol structure, sugar alcohols exhibit high hydrophilicity, which manifests as stronger hygroscopicity. While counteracting the affinity between gelatin protein and the packaging material, sugar alcohols tend to increase the surface stickiness of the gel composition. By maintaining a specific ratio between gelatin and the sugar alcohol, the present application not only reduces the affinity between gelatin protein and the packaging material but also controls the hygroscopicity of the sugar alcohol, thereby lowering the surface stickiness of the gel composition.

When a lipid soluble component is introduced into the gel composition, the molecular distribution between the aqueous and oil phases may affect the texture of the gel composition. In the gel composition provided by the present application, gelatin may serve both as a gelling agent and as an emulsifier. The gelling agent functions to provide shaping and textural support, while the emulsifier acts to stabilize and achieve equilibrium between immiscible systems. Additionally, the sugar alcohol and gelatin exhibit a certain synergistic effect in enhancing cohesiveness and emulsification performance. Their combination not only improves cohesiveness to reduce surface adhesion but also enhances the stability of the oil-containing gel composition. Even under high oil-loading conditions, a balanced and stable state between the oil and aqueous phases may be maintained. Thus, by introducing gelatin and a sugar alcohol, controlling their content and ratio in the gel composition, and regulating the pH value of the gel composition to maintain a certain difference from the isoelectric point of the gelatin, the present application achieves the effects of stabilizing and solidifying oils while improving the surface stickiness of the gel composition.

On the other hand, since gelatin is composed of protein and the charged groups in its structure affect its isoelectric point, the applicant has found that it tends to cause protein flocculation when the pH of the gel composition overlaps with the isoelectric point of the gelatin, thereby affecting the surface stickiness and gel strength of the gel composition. Therefore, the present application further specifies that the isoelectric point of the gelatin should be greater than the pH of the gel composition.

As described above, by introducing gelatin and a sugar alcohol, controlling their content and ratio in the gel composition, and regulating the pH value of the gel composition to maintain a certain difference from the isoelectric point of the gelatin, the present application balances the relationship among surface stickiness, cohesiveness, and packaging material affinity in the gel composition. This ensures that even a gel composition containing not less than 10% lipid soluble component may retain its complete form after removal from the packaging material, without adversely affecting the texture, hardness, moldability, or other properties of the gel composition.

In some implementations, the isoelectric point of the gelatin is 5.1 to 9.2.

In some implementations, the pH of the gel composition is 3.60 to 4.80.

In some implementations, an amount of alcoholic hydroxyl groups is not less than 4 in a chemical structure of the sugar alcohol.

In some implementations, the sugar alcohol includes at least one of xylitol, maltitol, sorbitol and erythritol.

In some implementations, the mass content of the lipid soluble component in the gel composition is 10% to 55%; and/or, the gel composition further includes a water-soluble active component, and the mass content of the water-soluble active component in the gel composition is 1% to 25%.

In some implementations, the fat-soluble component includes at least one of DHA algal oil, oil-soluble vitamins, evening primrose oil, arachidonic acid, linseed oil, caprylic/capric triglyceride, safflower seed oil, milk thistle seed oil, acer truncatum seed oil, walnut oil, phosphatidylserine, fish oil, coenzyme Q10, rice bran fatty alcohol, pumpkin seed oil, borage oil, sea buckthorn fruit oil, acetylsalicylic acid, lipophilic statins, antibiotics, naproxen, antihistamines, lutein, lutein ester, astaxanthin, and krill oil.

In some implementations, the water-soluble active component includes at least one of water-soluble vitamins, water-soluble minerals, ibuprofen, acetaminophen, caffeine, chlorphenamine maleate, and water-soluble statins.

In some implementations, the gel composition further includes an auxiliary agent, the auxiliary agent includes at least one of a pH adjuster, an antioxidant, an emulsifier, a humectant, a flavoring agent, and a coloring agent; the pH adjuster includes at least one of citric acid and salts thereof, malic acid and salts thereof, lactic acid, and tartaric acid.

In some implementations, the humectant includes glycerol.

In some implementations, the pH adjuster includes at least one of citric acid and salts thereof, malic acid and salts thereof, lactic acid, and tartaric acid.

In some implementations, the antioxidant includes at least one of tea polyphenols (TP), tocopherols, flavonoids (such as rosemary extract), butylated hydroxyanisole (BHA), butylated hydroxytoluene (BHT), tert-butylhydroquinone (TBHQ), and ascorbic acid compounds.

In some implementations, the emulsifier includes at least one of a phospholipid emulsifier, modified starch, gum, emulsified pectin, cholesterol, lanolin, saponin, and polysaccharide emulsifiers.

In some implementations, the flavoring agent includes at least one of natural or synthetic flavors, natural spices, fruit and vegetable juices, and fruit powders.

According to a second aspect of the present application, a product containing the gel composition is provided.

According to a third aspect of the present application, use of the aforementioned gel composition or product in food, pharmaceuticals, or cosmetics is provided.

In some implementations, the food is a health food.

### BRIEF DESCRIPTION OF THE DRAWINGS

FIG. 1 shows the gel composition provided in Example 1 of the present application and its adhesion situation after tearing open the packaging;
FIG. 2 shows the gel composition provided in Comparative Example 1 of the present application and its adhesion situation after tearing open the packaging.

### DETAILED DESCRIPTION

For a better understanding of the technical solutions of the present application by those skilled in the art, the technical solutions in the examples of the present application are clearly and completely described and discussed below. Obviously, the examples described herein are only some of the examples of the present application but not all of them. Based on the embodiments in the present application, all other embodiments obtained by those skilled in the art without creative efforts should fall within the scope of protection of the present application.

### Example 1

### 1. Materials Required for Preparation of Gel Composition

The raw materials required for the present example are shown in Table 1, with the remaining portion being water.

**Table 1. Formulation required for the gel composition prepared in the present Example**

| Component | Material | Mass Percentage |
|---|---|---|
| / | gelatin (isoelectric point: 9.0) | 6% |
| sugar alcohol | xylitol | 20% |
| | maltitol syrup (content: 55%) | 18% |
| lipid component | DHA algal oil | 35% |
| pH adjuster | citric acid monohydrate | 0.65% |
| | sodium citrate dihydrate | 0.2% |
| humectant | glycerol | 2% |
| flavoring agent | orange flavor | 2% |
| / | water | Balance |

### 2. Method for Preparing the Gel Composition

S1. The specified amounts of water, glycerol, and maltitol syrup were weighed and uniformly mixed in a beaker. Gelatin was then added, and the mixture was heated at 65 to 75°C until complete dissolution of the colloid was achieved. Xylitol, citric acid monohydrate, and sodium citrate dihydrate were subsequently added and stirred until completely dissolved. The mixture was maintained at temperature for later use, yielding the aqueous phase.

S2. The specified amount of DHA algal oil was weighed into a beaker, and the specified amount of orange flavor was added, yielding the oil phase.

S3. The aqueous phase was subjected to shear in a homogenizing shear machine. The oil phase was gradually added to the aqueous phase under shear homogenization, resulting in a gel composition with a pH of 4.0.

### Example 2

The present example refers to the formulation and method provided in Example 1 for preparing the gel composition. The difference from Example 1 is that the gelatin used in the present example had an isoelectric point of 5.04. Except for the aforementioned difference, the operational steps for preparing the gel composition in the present example were strictly consistent with those of Example 1.

### Example 3

The present example refers to the formulation and method provided in Example 1 for preparing the gel composition. The difference from Example 1 is that the gelatin used in the present example had an isoelectric point of 5.1. Except for the aforementioned difference, the operational steps for preparing the gel composition in the present example were strictly consistent with those of Example 1.

### Example 4

The present example refers to the formulation and method provided in Example 1 for preparing the gel composition. The difference from Example 1 is that the gelatin used in the present example had an isoelectric point of 9.2. Except for the aforementioned difference, the operational steps for preparing the gel composition in the present example were strictly consistent with those of Example 1.

### Example 5

The present example refers to the formulation and method provided in Example 1 for preparing the gel composition. The difference from Example 1 is that the pH of the gel composition was adjusted to 3.51 by modifying the content of the pH adjuster during preparation. Except for the aforementioned difference, the operational steps for preparing the gel composition in the present example were strictly consistent with those of Example 1.

### Example 6

The present example refers to the formulation and method provided in Example 1 for preparing the gel composition. The difference from Example 1 is that the pH of the gel composition was adjusted to 4.2 by modifying the content of the pH adjuster during preparation. Except for the aforementioned difference, the operational steps for preparing the gel composition in the present example were strictly consistent with those of Example 1.

### Example 7

The present example refers to the formulation and method provided in Example 1 for preparing the gel composition. The difference from Example 1 is that the pH of the gel composition was adjusted to 5.02 by modifying the content of the pH adjuster during preparation. Except for the aforementioned difference, the operational steps for preparing the gel composition in the present example were strictly consistent with those of Example 1.

### Example 8

The present example refers to the formulation and method provided in Example 1 for preparing the gel composition. The difference from Example 1 is that maltitol syrup was replaced with an equal mass fraction of xylitol during preparation. Except for the aforementioned difference, the operational steps for preparing the gel composition in the present example were strictly consistent with those of Example 1.

### Example 9

The present example refers to the formulation and method provided in Example 1 for preparing the gel composition. The difference from Example 1 is that xylitol was replaced with an equal mass fraction of maltitol syrup during preparation. Except for the aforementioned difference, the operational steps for preparing the gel composition in the present example were strictly consistent with those of Example 1.

### Example 10

The present example refers to the formulation and method provided in Example 1 for preparing the gel composition. The difference from Example 1 is that maltitol syrup was replaced with an equal mass fraction of sorbitol during preparation, and xylitol was replaced with an equal mass fraction of erythritol during preparation. Except for the aforementioned difference, the operational steps for preparing the gel composition in the present example were strictly consistent with those of Example 1.

### Example 11

The present example refers to the formulation and method provided in Example 1 for preparing the gel composition. The difference from Example 1 is that the content of gelatin was set to 4% (adjusted by varying the amount of water as the balance) during preparation. Except for the aforementioned difference, the operational steps for preparing the gel composition in the present example were strictly consistent with those of Example 1.

### Example 12

The present example refers to the formulation and method provided in Example 1 for preparing the gel composition. The difference from Example 1 is that the content of gelatin was set to 8.5% (adjusted by varying the amount of water as the balance) during preparation. Except for the aforementioned difference, the operational steps for preparing the gel composition in the present example were strictly consistent with those of Example 1.

### Example 13

The present example refers to the formulation and method provided in Example 1 for preparing the gel composition. The difference from Example 1 is that the content of gelatin was set to 13% (adjusted by varying the amount of water as the balance) during preparation. Except for the aforementioned difference, the operational steps for preparing the gel composition in the present example were strictly consistent with those of Example 1.

### Example 14

The present example refers to the formulation and method provided in Example 1 for preparing the gel composition. The difference from Example 1 is that the mass ratio of gelatin to sugar alcohol was set to 0.12 (while the total mass percentage of gelatin and sugar alcohol, as well as the mass ratio between maltitol syrup and xylitol, remained unchanged) during preparation. Except for the aforementioned difference, the operational steps for preparing the gel composition in the present example were strictly consistent with those of Example 1.

### Example 15

The present example refers to the formulation and method provided in Example 1 for preparing the gel composition. The difference from Example 1 is that the mass ratio of gelatin to sugar alcohol was set to 0.4 (while the total mass percentage of gelatin and sugar alcohol, as well as the mass ratio between maltitol syrup and xylitol, remained unchanged) during preparation. Except for the aforementioned difference, the operational steps for preparing the gel composition in the present example were strictly consistent with those of Example 1.

### Example 16

The present example refers to the formulation and method provided in Example 1 for preparing the gel composition. The difference from Example 1 is that a water-soluble active component, a coloring agent, and an emulsifier were additionally incorporated during preparation, with the specific formulation detailed in Table 2. Except for the aforementioned differences, the operational steps for preparing the gel composition in the present example were strictly consistent with those of Example 1.

**Table 2. Formulation required for the gel composition prepared in Example 16**

| Component | Material | Mass Percentage |
|---|---|---|
| / | gelatin (isoelectric point: 9.0) | 6% |
| sugar alcohol | xylitol | 20% |
| | maltitol syrup (Content: 55%) | 18% |
| water-soluble active component | vitamin C | 3% |
| lipid soluble component | DHA algal oil | 35% |
| pH adjuster | citric acid monohydrate | 0.65% |
| | sodium citrate dihydrate | 0.2% |
| humectant | glycerol | 2% |
| flavoring agent | orange flavor | 2% |
| coloring agent | natural annatto | 0.01% |
| emulsifier | phospholipid | 0.05% |
| / | water | Balance |

### Comparative Example 1

The present comparative example refers to the formulation and method provided in Example 1 for preparing the gel composition. The difference from Example 1 is that the pH of the gel composition was adjusted to be identical to the isoelectric point of the gelatin by modifying the content of the pH adjuster during preparation. Except for the aforementioned difference, the operational steps for preparing the gel composition in the present comparative example were strictly consistent with those of Example 1.

### Comparative Example 2

The present comparative example refers to the formulation and method provided in Example 1 for preparing the gel composition. The difference from Example 1 is that the pH of the gel composition was adjusted to deviate excessively from the isoelectric point of the gelatin, specifically achieving I - H = 5.3, by modifying the content of the pH adjuster during preparation. Except for the aforementioned difference, the operational steps for preparing the gel composition in the present comparative example were strictly consistent with those of Example 1.

### Comparative Example 3

The present comparative example refers to the formulation and method provided in Example 1 for preparing the gel composition. The difference from Example 1 is that the content of gelatin was set to 3% (adjusted by varying the amount of water as the balance) during preparation. Except for the aforementioned difference, the operational steps for preparing the gel composition in the present comparative example were strictly consistent with those of Example 1.

### Comparative Example 4

The present comparative example refers to the formulation and method provided in Example 1 for preparing the gel composition. The difference from Example 1 is that the content of gelatin was set to 14% (adjusted by varying the amount of water as the balance) during preparation. Except for the aforementioned difference, the operational steps for preparing the gel composition in the present comparative example were strictly consistent with those of Example 1.

### Comparative Example 5

The present comparative example refers to the formulation and method provided in Example 1 for preparing the gel composition. The difference from Example 1 is that the mass ratio of gelatin to sugar alcohol was set to 0.1 (while the total mass percentage of gelatin and sugar alcohol, as well as the mass ratio between maltitol syrup and xylitol, remained unchanged) during preparation. Except for the aforementioned difference, the operational steps for preparing the gel composition in the present comparative example were strictly consistent with those of Example 1.

### Comparative Example 6

The present comparative example refers to the formulation and method provided in Example 1 for preparing the gel composition. The difference from Example 1 is that the mass ratio of gelatin to sugar alcohol was set to 0.42 (while the total mass percentage of gelatin and sugar alcohol, as well as the mass ratio between maltitol syrup and xylitol, remained unchanged) during preparation. Except for the aforementioned difference, the operational steps for preparing the gel composition in the present comparative example were strictly consistent with those of Example 1.

### Comparative Example 7

The present comparative example refers to the formulation and method provided in Example 1 for preparing the gel composition. The difference from Example 1 is that no sugar alcohol was incorporated during preparation. Specifically, the formulation for this comparative example is detailed in Table 3. Except for the aforementioned difference, the operational steps for preparing the gel composition in the present comparative example were strictly consistent with those of Example 1.

**Table 3. Formulation required for the gel composition prepared in Comparative Example 7**

| Component | Material | Mass Percentage |
|---|---|---|
| / | gelatin (isoelectric point: 9.0) | 6% |
| lipid soluble component | DHA algal oil | 35% |
| pH adjuster | citric acid monohydrate | 0.65% |
| | sodium citrate dihydrate | 0.2% |
| humectant | glycerol | 2% |
| flavoring agent | orange flavor | 2% |
| / | water | Balance |

### Test Example

### 1. Test Subject

The gel compositions prepared in Examples 1 to 16 and Comparative Examples 1 to 7.

### 2. Test Method

(1) Cohesiveness, Gel Hardness, and Adhesiveness: A texture analyzer was used to perform TPA tests on the test subjects. The TPA test method was selected, and the deformation degree value was set. The sample was placed on the test platform, and the test probe performed two compressions on the sample. The test results were converted into various physical parameter data through a force sensor. Each example was tested repeatedly with 10 samples, and the average value was taken and recorded.

The adhesion performance corresponding to cohesiveness, gel hardness, and adhesiveness was judged based on experimentally accumulated data:
Cohesiveness: The lower the value, the lower the cohesiveness. Typically, when ≤0.65, the adhesion risk significantly increases.
Gel hardness: The lower the value, the lower the gel strength. Typically, when ≤6500, the adhesion risk significantly increases.
Adhesiveness: The larger the absolute value, the greater the adhesiveness. Typically, when the absolute value ≥5, the adhesion risk significantly increases. The above ranges are not fixed, as adhesion performance is a comprehensive result of cohesiveness, gel hardness, and adhesiveness.

(2) Actual Adhesion Performance (Surface Stickiness): The gel composition was poured into blisters and sealed. After natural cooling and molding, it was stored at 22 to 25°C for 24 hours. The packaging was then opened, and the adhesion situation of the sample was observed. "O" indicates that the sample retained its complete appearance after opening the packaging, while "X" indicates the opposite.

(3) pH of the Gel Composition: 99 g of purified water at 60 to 65°C was weighed, and 1.000 g of the sample was added to the purified water. The mixture was stirred until the sample was completely dissolved to obtain an emulsion. The pH of the emulsion was measured using a pH meter, and the data were recorded.

(4) Isoelectric Point: The corresponding test was performed according to the method of GB 6783, and the relevant data were recorded. Gelatin with specific values was selected.

### 3. Test Results and Analysis

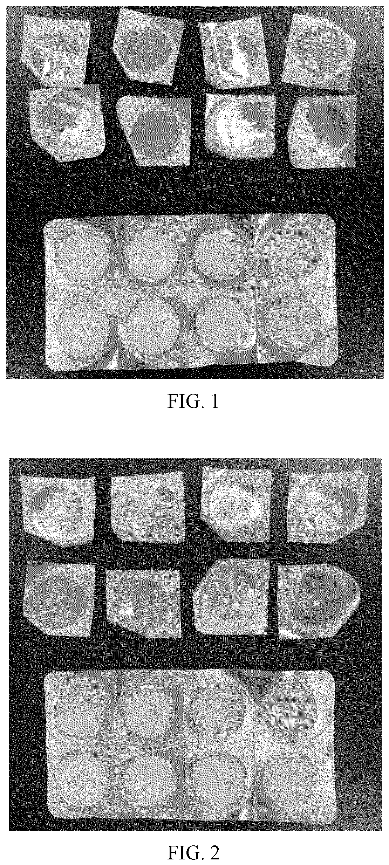

The test data for this test example are shown in Table 4. From FIG. 1 and FIG. 2, it was observed that the gel composition provided in Example 1 did not adhere to the packaging material after tearing, whereas the gel composition provided in Comparative Example 1 adhered to the packaging material.

Based on the data from Examples 1 to 7 and Comparative Examples 1 to 2, it was determined that the isoelectric point of the gelatin and the pH of the gel composition synergistically affect the surface stickiness of the gel composition. This is because, in the present application, gelatin functions both as a gelling agent and an emulsifier, providing shaping and textural support for the gel composition, as well as stabilizing and solidifying oils. When the pH of the gel composition overlaps with the isoelectric point of the gelatin, protein flocculation occurs in the gelatin, which not only reduces the cohesiveness and increases the adhesiveness of the gel composition but also adversely affects the gel strength, molding, and emulsification performance of the gel composition. Furthermore, by comparing the data, it was found that the cohesiveness decreases more significantly, and the surface stickiness increases when the pH of the gel composition is too low or the isoelectric point of the gelatin is too low.

By comparing the data from Example 1 and Examples 8 to 10, it was observed that the type and combination of sugar alcohols affect the cohesiveness, hardness, and adhesiveness of the gel composition. This is because the type of sugar alcohol influences the synergistic effect between sugar alcohols and gelatin in terms of emulsification and cohesiveness, as sugar alcohols may counteract the affinity between gelatin protein and packaging materials.

Based on the data from Examples 11 to 15 and Comparative Examples 3 to 7, it was concluded that the content of gelatin and sugar alcohols directly affects the surface stickiness of the gel composition. As the gelatin content increases, the cohesiveness also increases, thereby improving the surface stickiness of the gel composition. However, when the gelatin content is too high, the affinity of the protein in the gelatin for the packaging material also increases, leading to an increase in the surface stickiness of the gel composition and making it difficult to obtain an intact gel composition when the packaging material is opened. The polyhydroxyl alcohol structure of sugar alcohols results in high hydrophilicity and stronger hygroscopicity when the sugar alcohol content is too high, which conversely increases the surface stickiness of the gel composition.

**Table 4. Test Results of the Present Test Example**

| Group | I | H | Surface Stickiness | Cohesiveness | Gel Hardness /g | Adhesiveness /g·sec |
|---|---|---|---|---|---|---|
| Example 1 | 9.0 | 4.0 | O | 0.82 | 7240 | -3.3 |
| Example 2 | 5.04 | 4.0 | X | 0.52 | 7570 | -2.8 |
| Example 3 | 5.1 | 4.0 | O | 0.79 | 7460 | -3.5 |
| Example 4 | 9.2 | 4.0 | O | 0.80 | 6620 | -3.2 |
| Example 5 | 9.0 | 3.51 | X | 0.53 | 7130 | -3.7 |
| Example 6 | 9.0 | 4.2 | O | 0.85 | 7130 | -3.5 |
| Example 7 | 9.0 | 5.02 | X | 0.61 | 7250 | -3.6 |
| Example 8 | 9.0 | 4.0 | O | 0.81 | 7310 | -2.9 |
| Example 9 | 9.0 | 4.0 | O | 0.77 | 7160 | -3.7 |
| Example 10 | 9.0 | 4.0 | O | 0.83 | 7200 | -3.5 |
| Example 11 | 9.0 | 4.0 | O | 0.75 | 6280 | -1.8 |
| Example 12 | 9.0 | 4.0 | O | 0.87 | 8110 | -4.7 |
| Example 13 | 9.0 | 4.0 | O | 0.89 | 9040 | -5.2 |
| Example 14 | 9.0 | 4.0 | O | 0.78 | 6720 | -2.1 |
| Example 15 | 9.0 | 4.0 | O | 0.88 | 8470 | -4.9 |
| Example 16 | 9.0 | 4.0 | O | 0.82 | 7150 | -2.3 |
| Comparative Example 1 | 9.0 | 9.0 | X | 0.54 | 6270 | -3.3 |
| Comparative Example 2 | 9.0 | 3.7 | X | 0.57 | 7160 | -3.7 |
| Comparative Example 3 | 9.0 | 4.0 | X | 0.48 | 5640 | -1.5 |
| Comparative Example 4 | 9.0 | 4.0 | X | 0.90 | 9270 | -10.2 |
| Comparative Example 5 | 9.0 | 4.0 | X | 0.58 | 6270 | -3.4 |
| Comparative Example 6 | 9.0 | 4.0 | X | 0.81 | 8840 | -6.3 |
| Comparative Example 7 | 9.0 | 4.0 | X | 0.60 | 6530 | -4.8 |

The above examples are provided solely to illustrate the technical solutions of the present application and shall not be construed as limiting the scope of protection thereof. Although the present application has been described in detail with reference to preferred examples, ordinary technical personnel in the field should understand that modifications or equivalent replacements to the technical solutions of the present application may be made without departing from the spirit and scope of the technical solutions.

## Claims

1. A gel composition, comprising gelatin, a sugar alcohol, a lipid soluble component, and water;
wherein, in the gel composition, a mass content of the gelatin is 4% to 13%, and a mass content of the lipid soluble component is not less than 10%;
the weight ratio of gelatin to sugar alcohol is 0.12:1 to 0.4:1; and
an isoelectric point of the gelatin is I, and a pH of the gel composition after dilution to a mass concentration of 1% is H, and the following relationship within the gel composition is satisfied: 1 < I - H < 5.3.

2. The gel composition according to claim 1, wherein the isoelectric point of the gelatin is 5.1 to 9.2.

3. The gel composition according to claim 1 or 2, wherein the pH of the gel composition is 3.60 to 4.80.

4. The gel composition according to claim 1, wherein an amount of alcoholic hydroxyl groups is not less than 4 in a chemical structure of the sugar alcohol.

5. The gel composition according to claim 4, wherein the sugar alcohol comprises at least one of xylitol, maltitol, sorbitol and erythritol.

6. The gel composition according to claim 1, wherein the mass content of the lipid soluble component in the gel composition is 10% to 55%; and/or,
the gel composition further comprises a water-soluble active component, and the mass content of the water-soluble active component in the gel composition is 1% to 25%.

7. The gel composition according to claim 6, wherein the fat-soluble component comprises at least one of DHA algal oil, oil-soluble vitamins, evening primrose oil, arachidonic acid, linseed oil, caprylic/capric triglyceride, safflower seed oil, milk thistle seed oil, acer truncatum seed oil, walnut oil, phosphatidylserine, fish oil, coenzyme Q10, rice bran fatty alcohol, pumpkin seed oil, borage oil, sea buckthorn fruit oil, lutein, lutein ester, astaxanthin, and krill oil; and/or,
the water-soluble active component comprises at least one of water-soluble vitamins and water-soluble minerals.

8. The gel composition according to claim 6, wherein the fat-soluble component comprises at least one of acetylsalicylic acid, lipophilic statins, antibiotics, naproxen, and antihistamines; and/or,
the water-soluble active component comprises at least one of caffeine, ibuprofen, acetaminophen, chlorphenamine maleate, and water-soluble statins.

9. The gel composition according to claim 1, wherein the gel composition further comprises an auxiliary agent, the auxiliary agent comprises at least one of a pH adjuster, an antioxidant, an emulsifier, a humectant, a flavoring agent, and a coloring agent; the pH adjuster comprises at least one of citric acid and salts thereof, malic acid and salts thereof, lactic acid, and tartaric acid.

10. Use of the gel composition according to any one of claims 1 to 7 and claim 9 in a food or cosmetic.

11. A food, comprising the gel composition according to any one of claims 1 to 7 and claim 9.

12. A cosmetic, comprising the gel composition according to any one of claims 1 to 7 and claim 9.

13. Use of the gel composition according to any one of claims 1 to 9 in a pharmaceutical.

14. A pharmaceutical, comprising the gel composition according to any one of claims 1 to 9.
